# EUROPÄISCHE PATENTSCHRIFT

(11) **EP 3 258 833 B1**
(45) Veröffentlichungstag und Bekanntmachung des Hinweises auf die Patenterteilung: **11.07.2018**
(21) Anmeldenummer: 16724584.4
(22) Anmeldetag: 29.04.2016
(51) Int. Cl.: A61B 5/00, A61B 5/0476, A61B 5/0484, A61B 5/16

(54) **VERFAHREN ZUR BESTIMMUNG DER WAHRNEHMUNGSFÄHIGKEIT EINES PROBANDEN**
METHOD OF DETERMINING PERSON'S PERCEPTION CAPABILITY
METHODE POUR DETERMINER LA CAPACITE DE PERCEPTION D'UNE PERSONNE

(43) Veröffentlichungstag der Anmeldung: 27.12.2017
(73) Patentinhaber: Guger, Christoph, 4533 Piberbach (AT); Edlinger, Günter, 8020 Graz (AT)
(72) Erfinder: Guger, Christoph, 4533 Piberbach (AT); Edlinger, Günter, 8020 Graz (AT)
(74) Vertreter: Wildhack & Jellinek
(86) Internationale Anmeldenummer: PCT/AT2016/050117
(87) Internationale Veröffentlichungsnummer: WO 2017/185109

(56) Entgegenhaltungen:
- WO-A1-2014/052938
- WO-A1-2015/058223
- WO-A2-2012/104853
- J.R. KING ET AL: "Single-trial decoding of auditory novelty responses facilitates the detection of residual consciousness", NEUROIMAGE, Bd. 83, 1. Dezember 2013 (2013-12-01), Seiten 726-738, XP055123436, ISSN: 1053-8119, DOI: 10.1016/j.neuroimage.2013.07.013

## Beschreibung

Die Erfindung betrifft ein Verfahren zur Bestimmung der Wahrnehmungsfähigkeit oder Wahrnehmungsqualität eines Probanden, insbesondere eines Menschen, mittels eines Computer-Gehirn-Interface.

Aus dem Stand der Technik ist eine Vielzahl unterschiedlicher Methoden zur Bestimmung der Wahrnehmungsfähigkeit oder Wahrnehmungsqualität von Probanden bekannt, wobei sämtliche dieser Methoden unterschiedliche Arten von Stimuli auf den Probanden applizieren und anhand der Reaktion des Probanden in Form von Hirnströmen getestet wird, ob der betreffende Proband eine adäquate geistige Tätigkeit aufgrund der Stimuli zeigt oder nicht.

Im Stand der Technik besteht insbesondere das Problem, dass die Reaktion des Probanden oft nur schwer zu bewerten ist, und dass die Wahrnehmungsfähigkeit oder Wahrnehmungsqualität des Probanden während des Tests mitunter abnimmt oder ansteigt, insbesondere besteht bei Komapatienten das Problem, dass nicht klar ist, ob der betreffende Patient oder Proband gerade schläft. In diesem Zusammenhang besteht die Möglichkeit, dass nach einer Trainingsphase, während der der Proband wach ist, dieser einschläft und der erstellte Test eine sehr schlechte Wahrnehmungsfähigkeit des Probanden zeigt, während der Proband grundsätzlich jedoch durchaus wahrnehmungsfähig ist. Dies führt zu einer Fehlbeurteilung des geistigen Zustands und kann für den betreffenden Patienten oder Probanden mitunter erhebliche Konsequenzen haben, zumal derartige Tests nur äußerst selten durchgeführt werden.

Die Erfindung beseitigt diese Probleme bei einem Verfahren der eingangs genannten Art mit den Merkmalen des Patentanspruchs 1.

Die Erfindung Verfahren zur Bestimmung der Wahrnehmungsfähigkeit oder Wahrnehmungsqualität eines Probanden mittels eines Gehirn-Computer-Interface, das folgende Schritte aufweist:
a) Zumindest zwei voneinander unterscheidbare Arten von auf einen Probanden applizierbare Stimuli, insbesondere akustische, mechanische, elektrische oder optische Reize, werden vorgegeben, wobei der Proband insbesondere instruiert wird, bei Vorliegen eines bestimmten Stimulus eine bestimmte gedankliche Tätigkeit auszuführen, vorzugsweise zu zählen.
b) Eine Vielzahl von zeitlich hintereinander folgenden Stimuli wird auf den Probanden appliziert, die auf den Probanden applizierten Stimuli werden zu Blöcken zusammengefasst, wobei
   - die Blöcke eine Anzahl von zeitlich hintereinander folgenden auf den Probanden applizierten Stimuli umfassen, in denen jeweils ein Stimulus einer ersten Art an einer vorgegebenen Position des Blocks, sowie Stimuli von einer oder mehreren weiteren Arten an den übrigen Positionen des Blocks angeordnet sind,
   - nach jedem der Stimuli die Reaktion des Probanden festgestellt wird, indem die vom Stimulus bewirkten oder die mit diesem zeitlich in Zusammenhang stehenden EEG-Daten des Probanden ermittelt werden, und diese EEG-Daten dem jeweiligen Stimulus sowie dem jeweiligen Block an der betreffenden Position zugeordnet werden.
c) Aus den so ermittelten EEG-Daten werden Kalibrierdaten erstellt, indem eine Anzahl von ermittelten EEG-Daten und diesen EEG-Daten zugeordneten Stimuli zu Kalibrierblöcken zusammengesetzt werden, wobei in jeden einzelnen Kalibrierblock an einer Stelle jeweils die EEG-Daten des Probanden bei Applikation eines ersten Stimulus und an den übrigen Stellen die EEG-Daten des Probanden bei Applikation eines weiteren Stimulus zugeordnet werden, und
   - wobei mittels, Klassifikationsanalyse basierend auf ermittelten Kalibrierblöcken eine Klassifikationsfunktion ermittelt wird, die basierend auf EEG-Daten eines Blocks von Stimuli angibt, an welcher Position im jeweiligen Kalibrierblock sich der Stimulus der ersten Art befindet.
d) Die EEG-Daten werden einer Anzahl von aus den Blöcken ausgewählten Testblöcken der ermittelten Klassifikationsfunktion unterzogen und ein Klassifikationsergebnis wird ermittelt. Es wird untersucht wird, ob diejenige Position, an der sich der Stimulus erster Art im jeweiligen Testblock befindet, mit dem Klassifikationsergebnis übereinstimmt. Die Anzahl der Testblöcke für die eine Übereinstimmung erkannt wird, wird als Maß für die Wahrnehmungsfähigkeit eines Probanden zum Zeitpunkt der Aufnahme der weiteren EEG-Daten herangezogen.

Besonders vorteilhaft ist in diesem Zusammenhang, dass der Zeitpunkt, zu dem die einzelnen EEG-Daten ermittelt wurden, im Rahmen der Beurteilung der Wahrnehmungsfähigkeit oder Wahrnehmungsqualität nur von untergeordneter Rolle ist.

Eine besonders vorteilhafte Variante der Erfindung, mit dem eine hohe Durchmischung der einzelnen zu unterschiedlichen Zeitpunkten aufgenommenen EEG-Daten erreicht werden kann, sieht vor, dass basierend auf denselben ermittelten Blöcken die Schritte c) und d) mehrfach durchgeführt werden,
wobei bei jeder einzelnen Durchführung der Schritte c) und d) jeweils
- einzelne Blöcke insbesondere nach zufälligen Kriterien, in Schritt c) als Kalibrierblöcke ausgewählt werden, wobei diese Auswahl insbesondere zumindest 50% der Blöcke umfasst,
- aus den übrigen ermittelten Blöcken, insbesondere alle übrigen Blöcke, in Schritt d) als Testblöcke der Klassifkationsfunktion unterworfen werden,
- wobei bei jeder Durchführung der Schritte c) und d) jeweils ein separates Maß für die Wahrnehmungsfähigkeit oder Wahrnehmungsqualität ermittelt wird, und
dass ein weiteres Maß für die Wahrnehmungsfähigkeit oder Wahrnehmungsqualität durch Mittelwertbildung oder Aggregation der in den einzelnen Durchführungen erhaltenen separaten Maße ermittelt wird.

Eine vorteilhafte Vorgehensweise, die eine laufende Anpassung des betreffenden Wahrnehmungsmaßes ermöglicht und bei vollwahrnehmungsfähigen Patienten eine Konvergenz nach einem bestimmten Maximalwert zeigt, sieht vor, dass laufend Blöcke gemäß Schritt b) ermittelt werden, wobei nach der Aufnahme eines oder mehrerer Blöcke jeweils basierend auf den bislang ermittelten Blöcken), die Schritte c) und d) durchgeführt werden,
wobei bei jeder einzelnen Durchführung der Schritte c) und d) jeweils
- einzelne Blöcke insbesondere nach zufälligen Kriterien, in Schritt c) als Kalibrierblöcke ausgewählt werden, wobei diese Auswahl insbesondere zumindest 50% der Blöcke umfasst,
- aus den übrigen ermittelten Blöcken, insbesondere alle übrigen Blöcke, in Schritt d) als Testblöcke der Klassifkationsfunktion unterworfen werden,
- wobei bei jeder Durchführung der Schritte c) und d) jeweils ein separates Maß für die Wahrnehmungsfähigkeit oder Wahrnehmungsqualität ermittelt wird, und
dass untersucht wird, nach der Aufnahme wie vieler Blöcke das separate Maß für die Wahrnehmungsfähigkeit oder Wahrnehmungsqualität einen vorgegebenen Schwellenwert übersteigt und die Anzahl der Blöcke als weiteres Maß für die Wahrnehmungsfähigkeit oder Wahrnehmungsqualität ermittelt wird, und/oder dass der Mittelwert oder Median der einzelnen separates Maße als weiteres Maß für die Wahrnehmungsfähigkeit oder Wahrnehmungsqualität ermittelt wird.

Um eine möglichst vollständige Auswertung aller im Zuge der Aufnahme erhaltenen EEG-Daten zu erhalten, kann vorgesehen sein, dass einzelne Blöcke, insbesondere nach zufälligen Kriterien, in Schritt c) als Kalibrierblöcke ausgewählt werden, wobei diese Auswahl insbesondere zumindest 50% der Blöcke umfasst, - dass aus den übrigen ermittelten Blöcken, insbesondere alle übrigen Blöcke oder zumindest 10% der übrigen Blöcke, in Schritt d) als Testblöcke der Klassifkationsfunktion unterworfen werden und untersucht wird, ob diejenige Position, an der sich der Stimulus erster Art im jeweiligen Block befindet, mit dem Klassifikationsergebnis übereinstimmt.

Um die Bestimmung der Wahrnehmungsfähigkeit beim jeweiligen Probanden nachträglich einfach und schnell erneut vornehmen zu können, kann vorgesehen sein, dass nach der Bildung der Klassifikationsfunktion eine Anzahl von hintereinander folgenden weiteren Stimuli auf den Probanden appliziert wird, die entsprechend Schritt b) zu weiteren Blöcken zusammengefasst werden und dass für diese jeweils einzeln untersucht wird, ob diejenige Position, an der sich der Stimulus erster Art im jeweiligen weiteren Block befindet, mit dem Klassifikationsergebnis übereinstimmt und die Anzahl der weiteren Blöcke, für die eine Übereinstimmung erkannt wird, als Maß für die Wahrnehmungsfähigkeit eines Probanden zum Zeitpunkt der Aufnahme der weiteren EEG-Daten herangezogen wird.

Eine Steigerung des Maßes der Wahrnehmungsfähigkeit des Probanden kann zusätzlich erreicht werden, indem zumindest einer der Stimuli, insbesondere alle Stimuli oder wenigstens Stimuli der ersten Art, vibrotaktil auf den Probanden appliziert wird und dass bei Vorliegen eines einen Schwellenwert übersteigenden Klassifikationsergebnisses ein Aktivierungsstimulus auf den Probanden appliziert wird.

Hierbei kann in einer bevorzugten Ausführungsform der Erfindung insbesondere vorgesehen sein, dass der auf dem Probanden applizierte Aktivierungsstimulus in einer Applikation von funktioneller Elektrostimulation oder mit einer Orthese, einer Prothese oder einem Roboter auf einem Bereich des Körpers des Probanden besteht, mit der der Körper des Probanden an einer Stelle gereizt wird oder mit der ein Teil des Körpers des Probanden manipuliert wird, der vibrotaktil stimuliert wurde.

Eine weitere bevorzugte Ausführungsform der Erfindung, die bei einer Vielzahl von, auch nicht sehfähigen Patienten durchgeführt werden kann, sieht vor, dass die Menge der Arten von Stimuli durch unterschiedliche Töne, insbesondere mit unterschiedlicher Dauer, Frequenz und Lautstärke, in für einen Menschen hörbaren Frequenzen vorgegeben wird und der jeweilige Ton dem Probanden vorgespielt wird, oder dass die Menge der Arten von Stimuli Vibrationsbeaufschlagungen an unterschiedlichen Körperteilen und/oder mit unterschiedlicher Intensität und/oder Dauer umfasst, die dem Probanden mittels Vibrationseinheiten appliziert werden.

Eine weitere bevorzugte Ausführungsform der Erfindung, die bei einer Vielzahl von, auch gehörlosen Patienten durchgeführt werden kann, sieht vor, dass die Menge der Arten von Stimuli visuelle Reize für ein Auge oder beide Augen und/oder mit unterschiedlicher Intensität und/oder Dauer umfasst, die der Probanden mittels eines Bildschirms oder mittels Leuchtmitteln appliziert werden, oder
dass die Menge der Arten von Stimuli elektrische Reize an unterschiedlichen Körperteilen und/oder mit unterschiedlicher Intensität und/oder Dauer umfasst, die dem Probanden mittels elektrischer Stimulatoren appliziert werden.

Besonders gute Erkennbarkeit in den EEG-Daten kann erreicht werden, wenn der Proband abhängig vom jeweiligen Art von Stimulus eine der folgenden gedanklichen Tätigkeiten aufgetragen wird:
- Zählen oder Rechnen,
- Denken an Bewegungen von Körperteilen, insbesondere Extremitäten der rechten oder linken Körperhälfte, vorzugsweise der Arme oder Hände.

Eine besonders vorteilhafte Analyse der EEG-Daten kann erreicht werden, indem die Klassifikationsanzanalyse wie folgt duchgeführt werden:
- Diskriminanzanalyse, insbesondere linearer Diskriminanzanalyse,
- Support vector machines,
- neuronale Netzwerke.

Zur Bestimmung der Abhängigkeit der Wahrnehmungsfähigkeit und Wahrnehmungsqualität des Probanden von äußeren Einflüssen kann vorgesehen sein, dass nach der Bestimmung der Wahrnehmungsfähigkeit oder Wahrnehmungsqualität der Proband in verschiedene Zustände durch Kühlen oder Erwärmen des Körpers oder eines Teils des Körpers und/oder durch Medikation gebracht wird oder dass der Sauerstoffpartialdruck im Bereich des Probanden verändert wird und die Wahrnehmungsfähigkeit oder Wahrnehmungsqualität nach denselben Kriterien wiederholt wird und ein neues Maß für die Wahrnehmungsfähigkeit oder Wahrnehmungsqualität ermittelt wird, und das Maß und das neue Maß miteinander verglichen werden.

Zum selben Zweck kann vorteilhaft vorgesehen sein, dass nach der Bildung der Klassifikationsfunktion
- der Proband in verschiedene Zustände durch Kühlen oder Erwärmen des Körpers oder eines Teils des Körpers und/oder durch Medikation gebracht wird oder dass der Sauerstoffpartialdruck im Bereich des Probanden verändert wird, und
- eine Anzahl von hintereinander folgenden weiteren Stimuli auf den Probanden appliziert wird, die entsprechend Schritt b) zu weiteren Blöcken zusammengefasst werden und dass für diese jeweils einzeln untersucht wird, ob diejenige Position, an der sich der Stimulus erster Art im jeweiligen weiteren Block befindet, mit dem Klassifikationsergebnis übereinstimmt und die Anzahl der weiteren Blöcke, für die eine Übereinstimmung erkannt wird, als neues Maß für die Wahrnehmungsfähigkeit eines Probanden zum Zeitpunkt der Aufnahme der weiteren EEG-Daten herangezogen wird,
- und das Maß und das neue Maß miteinander verglichen werden.

Ein vorteilhaftes Feedback an den Probanden kann erreicht werden, indem die aufgenommenen EEG-Daten mittels zuvor durchgeführter Klassifikationsanalyse klassifiziert werden und dass bei Vorliegen eines ermittelten Ergebnisses aus der Klassifikation ein diesem Ergebnis zugeordneter Aktivierungsstimulus auf den Probanden appliziert wird.

Ein bevorzugtes Ausführungsbeispiel der Erfindung wird im Folgenden anhand der nachstehend genannten Zeichnungsfiguren dargestellt.

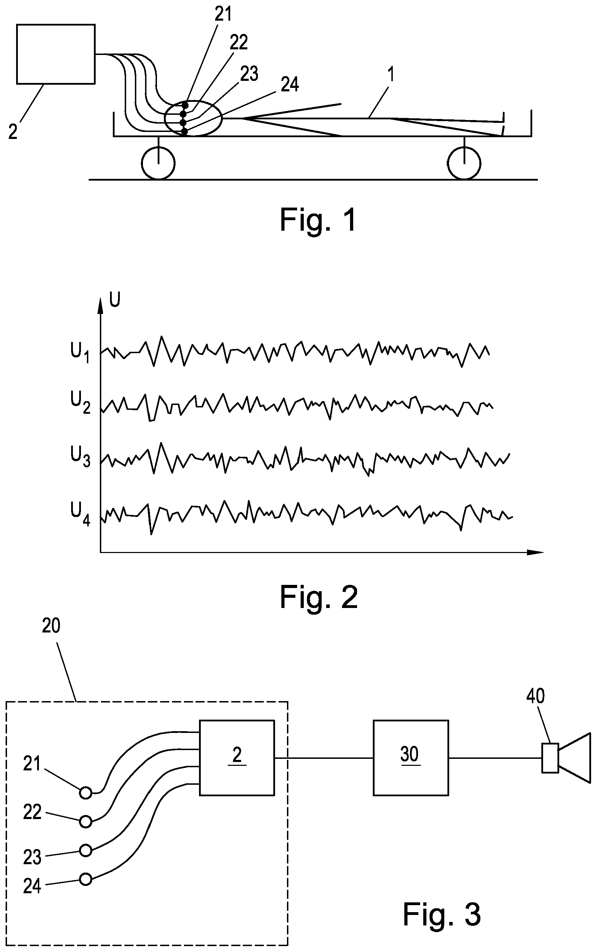

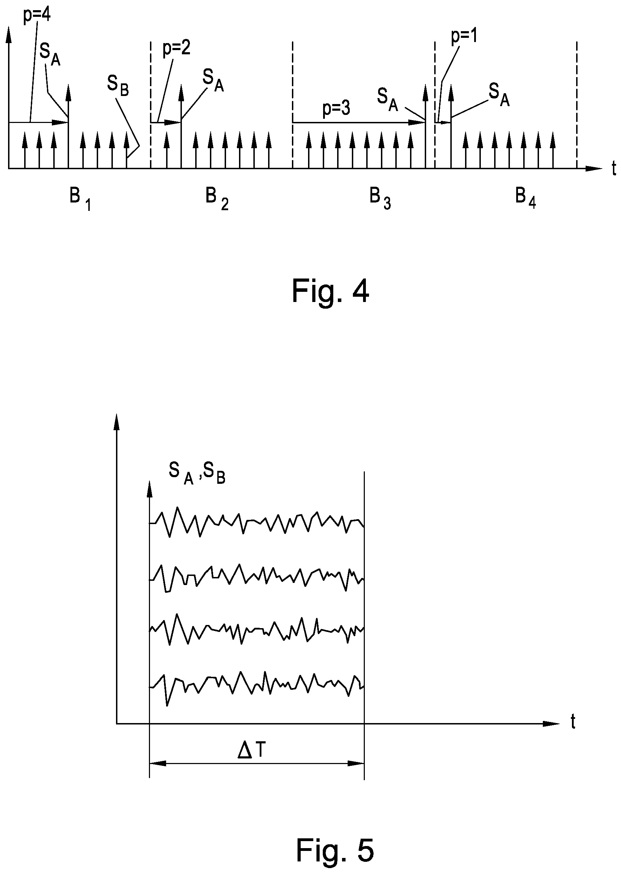

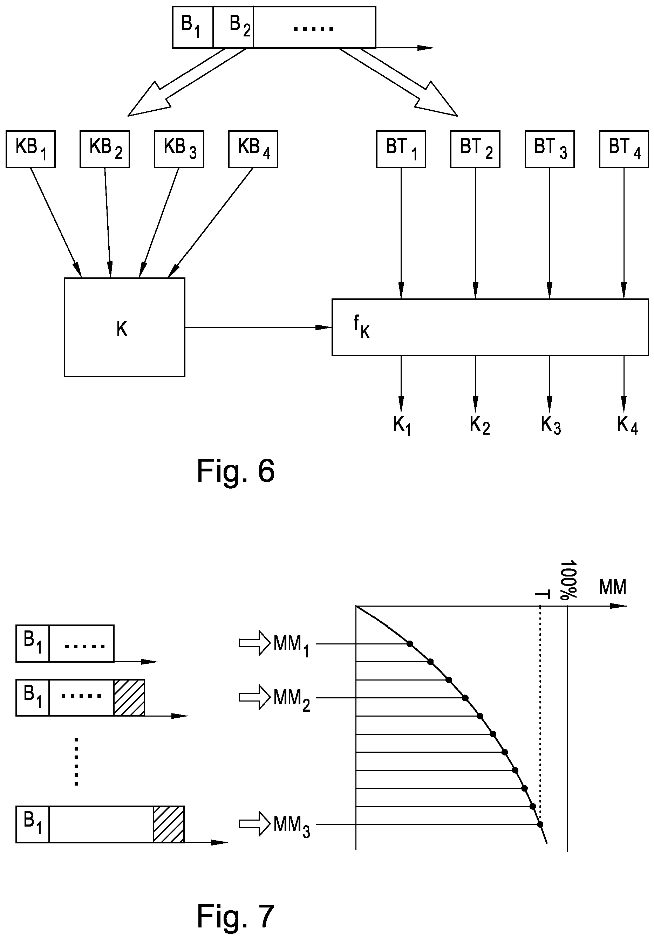

In **Fig. 1** ist ein Proband 1 in einem Bett liegend dargestellt. **Fig. 2** zeigt Spannungssignale, die mittels EEG-Elektroden ermittelt wurden. **Fig. 3** zeigt schematisch eine Anordnung zur Messung und Weiterverarbeitung von EEG-Daten. **Fig. 4** zeigt die zeitliche Abfolge der auf den Probanden applizierten Stimuli. **Fig. 5** zeigt die einem Stimulus zugeordneten EEG-Daten. **Fig. 6** zeigt schematisch das Vorgehen bei einer Ausführungsform eines erfindungsgemäßen Verfahrens. **Fig. 7** zeigt schematisch das Vorgehen bei einer bevorzugten Ausführungsform eines erfindungsgemäßen Verfahrens.

In **Fig. 1** ist ein Proband 1 in einem Bett liegend dargestellt. Auf dem Kopf des Probanden befindet sich eine Vielzahl von EEG-Elektroden 21-24, die allesamt an ein EEG-Verarbeitungsgerät 2 angeschlossen sind. Dieses wertet die einzelnen vom Probanden 1 abgegebenen Hirnströme aus und erstellt aus diesen Hirnströmen EEG-Daten U₁, U₂, U₃, U₄. Dabei können unterschiedliche Vorverarbeitungs- und Filterverfahren angewendet werden. Letztlich stehen EEG-Daten U₁, U₂, U₃, U₄ als Folge von gemessenen Spannungssignalen zur Verfügung, wobei jedes der Spannungssignale für eine Vielzahl von Zeitpunkten jeweils einen Spannungswert angibt (**Fig. 2**). Die Anordnung umfassend die einzelnen EEG-Elektroden 21-24 sowie die EEG-Messeinheit 2 stellt ein Gehirn-Computer-Interface 20 dar. Dieses Gehirn-Computer-Interface 20 ist an eine Test- und Steuereinheit 30 (**Fig. 3**) angeschlossen, die einen im vorliegenden Ausführungsbeispiel der Erfindung einen Lautsprecher 40 ansteuert. Bei diesem Lautsprecher 40 kann es sich beispielsweise um einen Lautsprecher 40 handeln, der in einem Headset angeordnet ist, das dem Probanden 1 aufgesetzt wurde.

Im vorliegenden Ausführungsbeispiel befinden sich die einzelnen Stimuli S_{A}, S_{B} in vorgegebenen Abständen zueinander sowie zum jeweiligen Beginnzeitpunkt des Blocks B₁, B₂, B₃, B₄. Besonders vorteilhaft weisen sämtliche Stimuli S_{A}, S_{B} zum jeweils nachfolgenden Stimulus S_{A}, S_{B} den selben zeitlichen Abstand auf, sodass für den Probanden 1 nicht erkennbar ist, wann ein Block B₁, B₂, B₃, B₄ endet und der nachfolgende Block B₁, B₂, B₃, B₄ beginnt.

Vorteilhafterweise kann der Proband 1 auch instruiert werden bei Vorliegen eines bestimmten Stimulus eine bestimmte gedankliche Tätigkeit auszuführen, insbesondere zu zählen oder zu rechnen, an bestimmte Bewegungen von Körperteilen zu denken, insbesondere an die Bewegung der Extremitäten zu denken. Derartige vom Probanden 1 vorgenommene Gedanken können mittels des Gehirn-Computer-Interface 20 aufgenommen werden und in der Test- und Steuereinheit 30 analysiert werden.

Im vorliegenden Ausführungsbeispiel wird eine Vielzahl von zeitlich hintereinander folgenden Stimuli S_{A}, S_{B} auf dem Probanden 1 appliziert (**Fig. 4**). Die auf dem Probanden 1 applizierten Stimuli S_{A}, S_{B} werden zu Blöcken B₁, B₂, B₃, B₄ zusammengefasst. Jeder der Blöcke B₁, B₂, B₃, B₄ umfasst dabei eine konkrete, im vorliegenden Fall auf acht festgelegte, Anzahl von hintereinander folgenden auf dem Probanden 1 applizierten Stimuli S_{A}, S_{B} und dabei befindet sich in jedem der Blöcke B₁, B₂, B₃, B₄ jeweils ein Stimulus S_{A} der ersten Art an einer bestimmten Position in Bezug auf den jeweiligen Block B₁, B₂, B₃, B₄. An den anderen Stellen befinden sich Stimuli S_{B} einer zweiten Art von Blöcken.

Im vorliegenden Ausführungsbeispiel handelt es sich bei den Stimuli S_{A} der ersten Art um den Probanden 1 vorgespielte hohe Töne, bei Stimuli S_{B} der zweiten Art um den Probanden 1 vorgespielte tiefere Töne. Jeder Block B₁, B₂, B₃, B₄ umfasst im vorliegenden Ausführungsbeispiel jeweils einen hohen Ton S_{A} sowie sieben tiefe Töne S_{B}. Für jeden einzelnen Ton wird das in **Fig. 5** dargestellte Vorgehen näher durchgeführt: Nach jedem der Stimuli S_{A}, S_{B} wird die Reaktion des Probanden 1 in Form von EEG-Daten U₁, U₂, U₃, U₄ festgestellt, indem die vom jeweiligen Stimulus S_{B} bewirkten oder die mit diesem zeitlichen Zusammenhang stehenden EEG-Daten U₁, U₂, U₃, U₄ des Probanden 1 ermittelt werden. Diese EEG-Daten U₁, U₂, U₃, U₄ werden dem jeweiligen Stimulus S_{A}, S_{B} sowie dem jeweiligen Block B₁, B₂, B₃, B₄ an der betreffenden Position zugeordnet. Im vorliegenden Ausführungsbeispiel werden für jeden der Stimuli S_{A}, S_{B} jeweils die nach dem Stimulus S_{A}, S_{B} innerhalb einer Zeitspanne von .-100 bis +700 ms aufgenommenen EEG-Daten U₁, U₂, U₃, U₄ herangezogen und den betreffenden Stimulus S_{A}, S_{B} zugeordnet. Allenfalls besteht auch die Möglichkeit, dass EEG-Daten U₁, U₂, U₃, U₄ herangezogen werden, die innerhalb einer bestimmten Zeitspanne vor Abgabe des Stimulus S_{A}, S_{B} aufgenommen wurden.

Aus der Gesamtheit der so gewonnenen EEG-Daten U₁, U₂, U₃, U₄ mit jeweils zugeordnetem Stimulus S_{A}, S_{B} werden Kalibrierdaten erstellt, in dem die einem Stimulus zugeordnete EEG-Daten U₁, U₂, U₃, U₄ gemeinsam mit dem jeweiligen Stimulus S_{A}, S_{B} zu Kalibrierblöcken KB₁, KB₂, KB₃, KB₄ zusammengesetzt werden (**Fig. 6**). Entsprechend den Blöcken werden die einzelnen Kalibrierblöcke KB₁, KB₂, KB₃, KB₄ jeweils derart zusammengesetzt, dass in jeweils einer Stelle jeweils die EEG-Daten U₁, U₂, U₃, U₄ des Probanden 1 bei Applikation eines ersten Stimulus S_{A} an deren übrigen Stellen EEG-Daten U₁, U₂, U₃, U₄ des Probanden 1 bei Applikation eines weiteres Stimulus S_{B} zugeordnet werden. Die Kalibrierblöcke KB₁, KB₂, KB₃, KB₄ können im Übrigen willkürlich zusammengesetzt werden, sodass es insbesondere nicht erforderlich ist, dass einzelne auf dem Probanden 1 applizierte Blöcke B₁, B₂, B₃, B₄ in ihrer Gesamtheit als Kalibrierblöcken KB₁, KB₂, KB₃, KB₄ ausgewählt werden. Die Erstellung der Kalibrierblöcke KB₁, KB₂, KB₃, KB₄ kann auch derart erfolgen, dass EEG-Daten U₁, U₂, U₃, U₄ und einzelne Stimuli nach zufälligen Kriterien ausgewählt und neuerlich zu Kalibrierblöcken KB₁, KB₂, KB₃, KB₄ zusammengesetzt werden wobei die Positionen p der Stimuli S_{A}, S_{B} und EEG-Daten U₁, U₂, U₃, U₄ variiert werden kann.

Für die Erstellung der Kalibrierblöcke KB₁, KB₂, KB₃, KB₄ können beispielsweise 80% der zur Verfügung stehenden EEG-Daten U₁, U₂, U₃, U₄ oder 80% der zur Verfügung stehenden aufgenommenen Blöcke B₁₅ B₂, B₃, B₄ herangezogen werden. In einem weiteren Schritt werden die einzelnen EEG-Daten U₁, U₂, U₃, U₄ einer Klassifikationsanalyse K unterzogen, wobei basierend auf den ermittelten Kalibrierblöcken KB₁, KB₂, KB₃, KB₄ eine Klassifikationsfunktion f_{K} ermittelt wird, die aufgrund von EEG-Daten U₁, U₂, U₃, U₄ eines Blocks B₁, B₂, B₃, B₄ oder Kalibrierblocks KB₁, KB₂, KB₃, KB₄ von EEG-Daten U₁, U₂, U₃, U₄ angibt, an welcher Position p im jeweiligen Block oder Kalibrierblock sich der Stimulus S_{A} der ersten Art befindet.

Dabei besteht vorteilhafterweise die Möglichkeit, dass die Klassifikationsanalyse auf die Kalibrierblöcke KB₁, KB₂, KB₃, KB₄ mit einem der im Folgenden dargestellten Klassifikationsverfahren durchgeführt wird:
- Diskriminanzanalyse, insbesondere linearer Diskriminanzanalyse,
- Support-Vector Machines
- neuronale Netzwerke.

Bei sämtlichen der vorstehend genannten Verfahren ist es möglich, ausgehend von dem bei Aufnahme eines Blocks B₁, B₂, B₃, B₄ ermittelten EEG-Daten U₁, U₂, U₃, U₄ eine Aussage darüber zu treffen, an welcher Position p innerhalb des jeweiligen Blocks B₁, B₂, B₃, B₄ sich der Stimulus S_{A} erster Art befindet.

Die übrigen ermittelten Blöcke BT₁, BT₂, BT₃, BT₄, insbesondere zumindest 10% der übrigen Blöcke BT₁, BT₂, BT₃, BT₄ oder die übrigen EEG-Daten U₁, U₂, U₃, U₄ werden als Testblöcke der ermittelten Klassifikationsfunktion f_{K} unterzogen. Es wird ein Klassifikationsergebnis K ermittelt, das angibt, ob diejenige Position p an der sich der Stimulus S_{A} erster Art im jeweiligen Testblock BT₁, BT₂, BT₃, BT₄ befindet mit dem Klassifikationsergebnis K übereinstimmt. Die Anzahl n der Testblöcke BT₁, BT₂, BT₃, BT₄, für die eine Übereinstimmung erkannt wird, wird als Maß M der Wahrnehmungsfähigkeit oder Wahrnehmungsqualität eines Probanden 1 zum Zeitpunkt der Aufnahme der EEG-Daten U₁, U₂, U₃, U₄ herangezogen.

Bei einer bevorzugten Ausführungsform der Erfindung besteht die Möglichkeit, die Schritte der Bildung einer Klassifikationsfunktion f_{K} sowie die Anwendung der jeweiligen Klassifikationsfunktion f_{K} auf die Testblöcke BT₁, BT₂, BT₃, BT₄ mehrfach durchzuführen. Hierbei wird für jede einzelne Durchführung dieser Schritte jeweils ein separates Maß M₁, ..., Mₙ für die Wahrnehmungsfähigkeit oder Wahrnehmungsqualität des Probanden 1 ermittelt. Aus diesen einzelnen Maßen M₁, ..., Mₙ kann ein weiteres Maß M* für die Wahrnehmungsfähigkeit oder Wahrnehmungsqualität ermittelt werden, beispielsweise durch Mittelwertbildung oder Aggregation der einzelnen ermittelten separaten Maße M₁, ..., Mₙ.

Ein weiteres Verfahren gemäß einer bevorzugten Ausführungsform der Erfindung hat den Vorteil, dass es auf eine stetig wachsende Menge von ermittelten bzw. aufgezeichneten Blöcken B₁, B₂, B₃, B₄ zugreifen kann (**Fig. 7**). Es werden hierbei laufend Blöcke von EEG-Daten U₁, U₂, U₃, U₄ ermittelt, wobei nach Aufnahme eines oder mehrerer Blöcke B₁, B₂, B₃, B₄ basierend auf den bislang ermittelten Blöcken B₁, B₂, B₃, B₄ jeweils separat eine Kalibrierfunktion f_{K} ermittelt wird und die übrigen ermittelten Blöcke B₁, B₂, B₃, B₄ oder ein Teil der übrigen ermittelten Blöcke der Klassifikationsfunktion f_{K} unterworfen wird. Auf diese Weise wird nach Aufnahme einer stetig wachsenden Anzahl von Blöcken B₁, B₂, B₃, B₄, jeweils ein separates Maß MM₁, ..., MMₙ, für die Wahrnehmungsfähigkeit oder Wahrnehmungsqualität des Probanden 1 ermittelt. Anschließend wird untersucht welche Anzahl n von Blöcken aufgenommen werden muss, bis das Maß MM₁, ..., MMₙ für die Wahrnehmungsfähigkeit oder Wahrnehmungsqualität einen vorgegebenen Schwellenwert T übersteigt.

Die Anzahl n der Blöcke, die erforderlich ist, dass das ermittelte Maß MM₁, ..., MMₙ den Schwellenwert T übersteigt, kann als weiters Maß M** für die Wahrnehmungsfähigkeit oder Wahrnehmungsqualität des Probanden 1 angesehen werden. Auch der Mittelwert oder Medial der einzelnen ermittelten Maße kann als weiteres Maß M*** für die Wahrnehmungsfähigkeit oder Wahrnehmungsqualität des Probanden 1 angesehen werden. Der Schwellenwert T kann vorteilhafterweise so festgelegt werden, dass der Proband 1 mehr als 90%, insbesondere mehr als 95%, der Blöcke B₁, B₂, B₃, B₄ richtig zuordnet bzw. für diesen Block B₁, B₂, B₃, B₄ die Position des Stimulus S_{A} erster Art im jeweiligen Block richtig feststellt.

Eine bevorzugte Ausführungsform der Erfindung, die mit allen vorstehend genannten Ausführungsvarianten der Erfindung kombinierbar ist, sieht vor, dass der Test, dem der Proband 1 unterzogen wurde, nach einer vorgegebenen Zeitspanne erneut durchgeführt wird. Besonders vorteilhaft an dieser Vorgehensweise ist, dass für den betreffenden Probanden bereits Klassifikationsfunktionen f_{K} vorliegen und es zu erwarten ist, dass der gleiche Proband 1 bei der Applikation derselben Stimuli S_{A}, S_{B} voraussichtlich dieselben Ergebnisse liefern wird, sodass eine aufwendige Bestimmung der Klassifikationsfunktion f_{K} nicht erforderlich ist. Dieses Vorgehen ist insbesondere dann von Vorteil, wenn die Zeit seit der Bestimmung der Klassifikationsfunktion f_{K} kurz ist.

Bei der erneuten Durchführung des vorstehend genannten Tests werden die aufgenommenen EEG-Daten U₁, U₂, U₃, U₄ und Stimuli S_{A}, S_{B} einander erneut zugeordnet und zu weiteren Blöcken B₁', B₂', B₃', B₄' zusammengefasst, wobei diese weiteren Blöcke B₁', B₂', B₃', B₄' denselben Aufbau aufweisen wie die Kalibrierblöcke KB₁, KB₂, KB₃, KB₄ sowie die im Zuge der Kalibrierung ermittelten Blöcke B₁, B₂, B₃, B₄. Für jeden der weiteren Blöcke B₁', B₂', B₃', B₄' wird jeweils einzeln untersucht, ob diejenige Position, an der sich der Stimulus S_{A}, ster Art im jeweiligen weiteren Block B₁', B₂', B₃', B₄' befindet, mit dem Klassifikationsergebnis K, das heißt dem Ergebnis der Anwendung der Klassifikationsfunktion f_{K} auf dem weiteren Block B₁', B₂', B₃', B₄' übereinstimmt. Die Anzahl der weiteren Blöcke B₁', B₂', B₃', B₄' für die eine Übereinstimmung erkannt wurde, wird bestimmt und als Maß M* für die Wahrnehmungsfähigkeit eines Probanden zum Zeitpunkt der Aufnahme der weiteren EEG-Daten U'₁, U'₂, U'₃, U'₄ herangezogen.

Bei sämtlichen genannten Ausführungsbeispielen der Erfindung ist es möglich, dass anstelle von unterschiedlich hohen Tönen auch Töne mit unterschiedlicher Dauer oder Lautstärke in für einen Menschen hörbaren Frequenzen vorgegeben werden. Alternativ besteht auch die Möglichkeit, dass der betreffende Proband 1 an unterschiedlichen Körperteilen mit Vibrationen beaufschlagt wird, wobei Intensität und Dauer der Vibrationen unterschiedlich ist und eine Unterscheidung zwischen erstem Stimulus und weiteren Stimuli S_{A}, S_{B} aufgrund unterschiedlicher Intensität oder Dauer der Vibrationsbeaufschlagungen möglich ist.

Alternativ besteht auch die Möglichkeit, dass die Menge der Arten von Stimuli S_{A}, S_{B} visuelle Reize für ein Auge oder beide Augen und/oder mit unterschiedlicher Intensität und/oder Dauer umfasst, die auf den Probanden 1 mittels eines Bildschirms oder mittels eines Leuchtmittels appliziert werden. Alternativ kann die Menge der Arten von Stimuli S_{A}, S_{B} auch elektrische Reize, die an unterschiedlichen Körperteilen und/oder mit unterschiedlicher Intensität und/oder unterschiedlicher Dauer ausgeübt werden, umfassen.

Sofern der Proband 1 mit vibrotaktilen Stimuli S_{A}, S_{B} beaufschlagt wurde, besteht in einer bevorzugten Ausführungsform der Erfindung die Möglichkeit, dass dem Probanden 1 nach erfolgreicher Feststellung einer einen Schwellenwert überschreitenden Wahrnehmungsfähigkeit oder Wahrnehmungsqualität in einer Applikation und funktioneller Elektrostimulation oder mit einer Orthese einer Prothese oder einem Roboter auf einem Bereich des Körpers des Probanden 1 besteht, mit der der Körper des Probanden 1 an einer Stelle gereizt wird oder mit einem Teil des Körpers des Probanden 1 manipuliert wird, der vibrotaktil stimuliert wurde.

Mit dieser Maßnahme kann dem Probanden ein Feedback geliefert werden. Die aufgenommenen EEG-Daten werden dabei mittels zuvor durchgeführter Klassifikationsanalyse klassifiziert. Bei Vorliegen eines ermittelten Ergebnisses aus der Klassifikation wird auf den Probanden ein diesem Ergebnis zugeordneter Aktivierungsstimulus appliziert wird.

In einigen Fällen kann es von Vorteil sein, wenn das vorstehend beschriebene Vorgehen wiederholt wird, wenn der Proband bestimmten Einflüssen ausgesetzt ist. Solche Einflüsse können etwa sein:
- Kühlen oder Erwärmen des Körpers oder eines Teils des Körpers des Probanden
- Medikation
- Änderung des Sauerstoffpartialdrucks im Bereich des Probanden

Der Test wird unter diesen Einflüssen erneut durchgeführt. Dies ermöglicht es, die Abhängigkeit der Wahrnehmungsfähigkeit des Probanden 1 in Abhängigkeit von den jeweiligen äußeren Einflüssen zu ermitteln.

Hierbei ist es nicht erforderlich, dass die Klassifikationsfunktion bei Vorliegen des äußeren Einflusses neu gebildet wird. Vielmehr kann, wie vorstehend beschrieben die Klassifikationsfunktion beibehalten werden. Lediglich die neu bei Vorliegen des äußeren Einflusses ermittelten weiteren Blöcke werden der Klassifikationsfunktion unterzogen. Auf diese Weise wird ein neues Maß für die Wahrnehmungsfähigkeit und Wahrnehmungsqualität gebildet und mit dem bisher ermittelten Maß für die Wahrnehmungsfähigkeit und Wahrnehmungsqualität verglichen.

## Patentansprüche

1. Verfahren zur Bestimmung der Wahrnehmungsfähigkeit oder Wahrnehmungsqualität eines Probanden (1) mittels eines Gehirn-Computer-Interface (20), wobei
a) zumindest zwei voneinander unterscheidbare Arten von auf einen Probanden (1) applizierbare Stimuli (S_{A}, S_{B}), insbesondere akustische, mechanische, elektrische oder optische Reize, vorgegeben werden, wobei der Proband (1) insbesondere instruiert wird, bei Vorliegen eines bestimmten Stimulus (S_{A}, S_{B}) eine bestimmte gedankliche Tätigkeit auszuführen, vorzugsweise zu zählen,
b) wobei eine Vielzahl von zeitlich hintereinander folgenden Stimuli (S_{A}, S_{B}) auf den Probanden (1) appliziert wird, und die auf den Probanden (1) applizierten Stimuli (S_{A}, S_{B}) zu Blöcken (B₁, B₂, B₃, B₄) zusammengefasst werden, wobei
- die Blöcke (B₁, B₂, B₃, B₄) eine Anzahl von zeitlich hintereinander folgenden auf den Probanden (1) applizierten Stimuli (S_{A}, S_{B}) umfassen, in denen jeweils ein Stimulus (S_{A}) einer ersten Art an einer vorgegebenen Position (p) des Blocks (B₁, B₂, B₃, B₄), sowie Stimuli (S_{B}) von einer oder mehreren weiteren Arten an den übrigen Positionen des Blocks (B₁, B₂, B₃, B₄) angeordnet sind,
- nach jedem der Stimuli (S_{A}, S_{B}) die Reaktion des Probanden (1) festgestellt wird, indem die vom Stimulus (S_{A}, S_{B}) bewirkten oder die mit diesem zeitlich in Zusammenhang stehenden EEG-Daten (U₁, U₂, U₃, U₄) des Probanden (1) ermittelt werden, und diese EEG-Daten (U₁, U₂, U₃, U₄) dem jeweiligen Stimulus (S_{A}, S_{B}) sowie dem jeweiligen Block an der betreffenden Position (p) zugeordnet werden,
c) wobei aus den so ermittelten EEG-Daten (U) Kalibrierdaten erstellt werden, indem eine Anzahl von ermittelten EEG-Daten (U₁, U₂, U₃, U₄) und diesen EEG-Daten (U₁, U₂, U₃, U₄) zugeordneten Stimuli (S_{A}, S_{B}) zu Kalibrierblöcken (KB₁, KB₂, KB₃, KB₄) zusammengesetzt werden, wobei in jeden einzelnen Kalibrierblock (KB₁, KB₂, KB₃, KB₄) an einer Stelle jeweils die EEG-Daten (U₁, U₂, U₃, U₄) des Probanden (1) bei Applikation eines ersten Stimulus (S_{A}, S_{B}) und an den übrigen Stellen die EEG-Daten (U₁, U₂, U₃, U₄) des Probanden (1) bei Applikation eines weiteren Stimulus (S_{A}, S_{B}) zugeordnet werden,
- wobei mittels, Klassifikationsanalyse basierend auf ermittelten Kalibrierblöcken (KB₁, KB₂, KB₃, KB₄) eine Klassifikationsfunktion (f_{K}) ermittelt wird, die basierend auf EEG-Daten (U₁, U₂, U₃, U₄) eines Blocks (B₁, B₂, B₃, B₄) von Stimuli (S_{A}, S_{B}) angibt, an welcher Position (p) im jeweiligen Kalibrierblock (KB₁, KB₂, KB₃, KB₄) sich der Stimulus (S_{A}, S_{B}) der ersten Art befindet, und
d) die EEG-Daten (U₁, U₂, U₃, U₄) einer Anzahl von aus den Blöcken ausgewählten Testblöcken (BT₁, BT₂, BT₃, BT₄) der ermittelten Klassifikationsfunktion (f_{K}) unterzogen werden und ein Klassifikationsergebnis (K) ermittelt wird, und untersucht wird, ob diejenige Position (p), an der sich der Stimulus (S_{A}) erster Art im jeweiligen Testblock (BT₁, BT₂, BT₃, BT₄) befindet, mit dem Klassifikationsergebnis (K) übereinstimmt, und die Anzahl der Testblöcke (BT₁, BT₂, BT₃, BT₄) für die eine Übereinstimmung erkannt wird oder ein davon abgeleitete Wert, als Maß (M) für die Wahrnehmungsfähigkeit eines Probanden (1) zum Zeitpunkt der Aufnahme der weiteren EEG-Daten (U₁, U₂, U₃, U₄) herangezogen wird.

2. Verfahren nach Anspruch 1, **dadurch gekennzeichnet, dass** basierend auf denselben ermittelten Blöcken (B₁, B₂, B₃, B₄) die Schritte c) und d) mehrfach durchgeführt werden, wobei bei jeder einzelnen Durchführung der Schritte c) und d) jeweils
- einzelne Blöcke(B₁, B₂, B₃, B₄), insbesondere nach zufälligen Kriterien, in Schritt c) als Kalibrierblöcke (KB₁, KB₂, KB₃, KB₄) ausgewählt werden, wobei diese Auswahl insbesondere zumindest 50% der Blöcke (B₁, B₂, B₃, B₄) umfasst,
- aus den übrigen ermittelten Blöcken, insbesondere alle übrigen Blöcke, in Schritt d) als Testblöcke (BT₁, BT₂, BT₃, BT₄) der Klassifkationsfunktion (f_{K}) unterworfen werden,
- wobei bei jeder Durchführung der Schritte c) und d) jeweils ein separates Maß für die Wahrnehmungsfähigkeit oder Wahrnehmungsqualität ermittelt wird, und
dass ein weiteres Maß (M') für die Wahrnehmungsfähigkeit oder Wahrnehmungsqualität durch Mittelwertbildung oder Aggregation der in den einzelnen Durchführungen erhaltenen separaten Maße (M₁, M₂, M₃, M₄) ermittelt wird.

3. Verfahren nach Anspruch 1 oder 2, **dadurch gekennzeichnet, dass** laufend Blöcke (B₁, B₂, B₃, B₄) gemäß Schritt b) ermittelt werden, wobei nach der Aufnahme eines oder mehrerer Blöcke (B₁, B₂, B₃, B₄) jeweils basierend auf den bislang ermittelten Blöcken (B₁, B₂, B₃, B₄), die Schritte c) und d) durchgeführt werden,
wobei bei jeder einzelnen Durchführung der Schritte c) und d) jeweils
- einzelne Blöcke (B₁, B₂, B₃, B₄), insbesondere nach zufälligen Kriterien, in Schritt c) als Kalibrierblöcke (KB₁, KB₂, KB₃, KB₄) ausgewählt werden, wobei diese Auswahl insbesondere zumindest 50% der Blöcke (B₁, B₂, B₃, B₄) umfasst,
- aus den übrigen ermittelten Blöcken (B₁, B₂, B₃, B₄), insbesondere alle übrigen Blöcke (B₁, B₂, B₃, B₄), in Schritt d) als Testblöcke (BT₁, BT₂, BT₃, BT₄) der Klassifkationsfunktion (f_{K}) unterworfen werden,
- wobei bei jeder Durchführung der Schritte c) und d) jeweils ein separates Maß (MM₁, ..., MMₙ) für die Wahrnehmungsfähigkeit oder Wahrnehmungsqualität ermittelt wird, und
dass untersucht wird, nach der Aufnahme wie vieler Blöcke (B₁, B₂, B₃, B₄) das separate Maß (MM₁, ..., MMₙ) für die Wahrnehmungsfähigkeit oder Wahrnehmungsqualität einen vorgegebenen Schwellenwert (T) übersteigt und die Anzahl der Blöcke (B₁, B₂, B₃, B₄) als weiteres Maß (M**) für die Wahrnehmungsfähigkeit oder Wahrnehmungsqualität ermittelt wird, und/oder dass der Mittelwert oder Median der einzelnen separates Maße (MM₁, ..., MMₙ) als weiteres Maß (M***) für die Wahrnehmungsfähigkeit oder Wahrnehmungsqualität ermittelt wird.

4. Verfahren nach einem der vorangehenden Ansprüche, **dadurch gekennzeichnet,**
- **dass** einzelne Blöcke (B₁, B₂, B₃, B₄), insbesondere nach zufälligen Kriterien, in Schritt c) als Kalibrierblöcke (KB₁, KB₂, KB₃, KB₄) ausgewählt werden, wobei diese Auswahl insbesondere zumindest 50% der Blöcke (B₁, B₂, B₃, B₄) umfasst,
- **dass** aus den übrigen ermittelten Blöcken (B₁, B₂, B₃, B₄), insbesondere alle übrigen Blöcke (B₁, B₂, B₃, B₄) oder zumindest 10% der übrigen Blöcke (B₁, B₂, B₃, B₄), in Schritt d) als Testblöcke (BT₁, BT₂, BT₃, BT₄) der Klassifkationsfunktion (f_{K}) unterworfen werden und untersucht wird, ob diejenige Position (p), an der sich der Stimulus (S_{A}) erster Art im jeweiligen Block befindet, mit dem Klassifikationsergebnis übereinstimmt.

5. Verfahren nach einem der vorangehenden Ansprüche, **dadurch gekennzeichnet, dass** nach der Bildung der Klassifikationsfunktion (f_{K}) eine Anzahl von hintereinander folgenden weiteren Stimuli (S_{A}, S_{B}) auf den Probanden (1) appliziert wird, die entsprechend Schritt b) zu weiteren Blöcken (B₁', B₂', B₃', B₄') zusammengefasst werden und dass für diese jeweils einzeln untersucht wird, ob diejenige Position (p), an der sich der Stimulus (S_{A}) erster Art im jeweiligen weiteren Block befindet, mit dem Klassifikationsergebnis (K) übereinstimmt und die Anzahl der weiteren Blöcke (B₁', B₂', B₃', B₄'), für die eine Übereinstimmung erkannt wird, als Maß für die Wahrnehmungsfähigkeit eines Probanden (1) zum Zeitpunkt der Aufnahme der weiteren EEG-Daten (U'₁, U'₂, U'₃, U'₄) herangezogen wird.

6. Verfahren nach einem der vorangehenden Ansprüche, **dadurch gekennzeichnet,**
- **dass** zumindest einer der Stimuli (S_{A}, S_{B}), insbesondere alle Stimuli (S_{A}, S_{B}) oder wenigstens Stimuli (S_{A}) der ersten Art, vibrotaktil auf den Probanden (1) appliziert wird,
- **dass** bei Vorliegen eines einen Schwellenwert übersteigenden Klassifikationsergebnisses ein Aktivierungsstimulus auf den Probanden (1) appliziert wird.

7. Verfahren nach Anspruch 6, **dadurch gekennzeichnet, dass** der auf dem Probanden (1) applizierte Aktivierungsstimulus in einer Applikation von funktioneller Elektrostimulation oder mit einer Orthese, einer Prothese oder einem Roboter auf einem Bereich des Körpers des Probanden (1) besteht, mit der der Körper des Probanden (1) an einer Stelle gereizt wird oder mit der ein Teil des Körpers des Probanden (1) manipuliert wird, der vibrotaktil stimuliert wurde.

8. Verfahren nach einem der Ansprüche 1 bis 5, **dadurch gekennzeichnet,**
- **dass** die Menge der Arten von Stimuli (S_{A}, S_{B}) durch unterschiedliche Töne, insbesondere mit unterschiedlicher Dauer, Frequenz und Lautstärke, in für einen Menschen hörbaren Frequenzen vorgegeben wird und der jeweilige Ton dem Probanden (1) vorgespielt wird, oder
- **dass** die Menge der Arten von Stimuli (S_{A}, S_{B}) Vibrationsbeaufschlagungen an unterschiedlichen Körperteilen und/oder mit unterschiedlicher Intensität und/oder Dauer umfasst, die dem Probanden (1) mittels Vibrationseinheiten appliziert werden.

9. Verfahren nach einem der vorangehenden Ansprüche, **dadurch gekennzeichnet, dass** die Menge der Arten von Stimuli (S_{A}, S_{B}) visuelle Reize für ein Auge oder beide Augen und/oder mit unterschiedlicher Intensität und/oder Dauer umfasst, die der Probanden (1) mittels eines Bildschirms oder mittels Leuchtmitteln appliziert werden, oder
dass die Menge der Arten von Stimuli (S_{A}, S_{B}) elektrische Reize an unterschiedlichen Körperteilen und/oder mit unterschiedlicher Intensität und/oder Dauer umfasst, die dem Probanden (1) mittels elektrischer Stimulatoren appliziert werden.

10. Verfahren nach einem der vorangehenden Ansprüche, **dadurch gekennzeichnet, dass** der Proband (1) abhängig vom jeweiligen Art von Stimulus (S_{A}, S_{B}) eine der folgenden gedanklichen Tätigkeiten aufgetragen wird:
- Zählen oder Rechnen,
- Denken an Bewegungen von Körperteilen, insbesondere Extremitäten der rechten oder linken Körperhälfte, vorzugsweise der Arme oder Hände.

11. Verfahren nach einem der vorangehenden Ansprüche, **dadurch gekennzeichnet, dass** die Klassifikationsanzanalyse wie folgt duchgeführt werden:
- Diskriminanzanalyse, insbesondere linearer Diskriminanzanalyse,
- Support vector machines,
- neuronale Netzwerke.

12. Verfahren nach einem der vorangehenden Ansprüchen, **dadurch gekennzeichnet, dass** nach der Bestimmung der Wahrnehmungsfähigkeit oder Wahrnehmungsqualität der Proband in verschiedene Zustände durch Kühlen oder Erwärmen des Körpers oder eines Teils des Körpers und/oder durch Medikation gebracht wird oder dass der Sauerstoffpartialdruck im Bereich des Probanden verändert wird und
die Wahrnehmungsfähigkeit oder Wahrnehmungsqualität nach denselben Kriterien wiederholt wird und ein neues Maß für die Wahrnehmungsfähigkeit oder Wahrnehmungsqualität ermittelt wird, und das Maß und das neue Maß miteinander verglichen werden.

13. Verfahren nach einem der vorangehenden Ansprüche, **dadurch gekennzeichnet, dass** nach der Bildung der Klassifikationsfunktion (f_{K})
- der Proband in verschiedene Zustände durch Kühlen oder Erwärmen des Körpers oder eines Teils des Körpers und/oder durch Medikation gebracht wird oder dass der Sauerstoffpartialdruck im Bereich des Probanden verändert wird, und
- eine Anzahl von hintereinander folgenden weiteren Stimuli (S_{A}, S_{B}) auf den Probanden (1) appliziert wird, die entsprechend Schritt b) zu weiteren Blöcken (B₁', B₂', B₃', B₄') zusammengefasst werden und dass für diese jeweils einzeln untersucht wird, ob diejenige Position (p), an der sich der Stimulus (S_{A}) erster Art im jeweiligen weiteren Block befindet, mit dem Klassifikationsergebnis (K) übereinstimmt und die Anzahl der weiteren Blöcke (B₁', B₂', B₃', B₄'), für die eine Übereinstimmung erkannt wird, als neues Maß für die Wahrnehmungsfähigkeit eines Probanden (1) zum Zeitpunkt der Aufnahme der weiteren EEG-Daten (U'₁, U'₂, U'₃, U'₄) herangezogen wird,
- und das Maß und das neue Maß miteinander verglichen werden.

14. Verfahren nach einem der vorangehenden Ansprüchen, **dadurch gekennzeichnet, dass** die aufgenommenen EEG-Daten mittels zuvor durchgeführter Klassifikationsanalyse klassifiziert werden und dass bei Vorliegen eines ermittelten Ergebnisses aus der Klassifikation ein diesem Ergebnis zugeordneter Aktivierungsstimulus auf den Probanden appliziert wird.

## Claims

1. A method for determining the perception capability or perception quality of a test subject (1) using a computer-brain interface (20), wherein:
a) at least two types of stimuli (S_{A}, S_{B}), applicable to a test subject (1), distinguishable from one another, especially acoustic, mechanical, electrical or optical stimuli, are presented, wherein the test subject (1) is especially instructed to perform a certain cognitive activity, preferably to count, when a certain stimulus (S_{A}, S_{B}) is present,
b) wherein a plurality of chronologically successive stimuli (S_{A}, S_{B}) is applied to the test subject (1), and the stimuli (S_{A}, S_{B}) applied to the test subject (1) are combined into blocks (B₁, B₂, B₃, B₄), wherein
- the blocks (B₁, B₂, B₃, B₄) comprise a number of chronologically successive stimuli (S_{A}, S_{B}) applied to the test subject (1), in which in each case a stimulus (S_{A}) of a first type is located at a predetermined position (p) of the block (B₁, B₂, B₃, B₄), and stimuli (S_{B}) of one or more different types are arranged at the other positions of the block (B₁, B₂, B₃, B₄),
- after each of the stimuli (S_{A}, S_{B}) the reaction of the test subject (1) is determined, in that the EEG data (U₁, U₂, U₃, U₄) of the test subject (1), provoked by the stimulus (S_{A}, S_{B}) or chronologically correlated with this, is determined, and these EEG data (U₁, U₂, U₃, U₄) are assigned to the respective stimulus (S_{A}, S_{B}) and the respective block at the position (p) in question,
c) wherein calibration data are from the EEG data (U) obtained in this way calibration data are compiled, in that a number of EEG data (U₁, U₂, U₃, U₄) and stimuli (S_{A}, S_{B}) assigned to these EEG data (U₁, U₂, U₃, U₄) are combined into calibration blocks (KB₁, KB₂, KB₃, KB₄) wherein in each individual calibration block (KB₁, KB₂, KB₃, KB₄) at one point in each case the EEG data (U₁, U₂, U₃, U₄) of the test subject (1) on application of a first stimulus (S_{A}, S_{B}), and at the other points the EEG data (U₁, U₂, U₃, U₄) of the test subject (1) upon application of an additional stimulus (S_{A}, S_{B}) are assigned,
- wherein by means of classification analysis based on the determined calibration blocks (KB₁, KB₂, KB₃, KB₄) a classification function (f_{K}) is determined, which based on EEG data (U₁, U₂, U₃, U₄) of a block (B₁, B₂, B₃, B₄) of stimuli (S_{A}, S_{B}) indicates at which position (p) in the respective calibration block (KB₁, KB₂, KB₃, KB₄) the stimulus (S_{A}, S_{B}) of the first type is located, and
d) the EEG data (U₁, U₂, U₃, U₄) of a number of test blocks (BT₁, BT₂, BT₃, BT₄) selected from the blocks are subjected to the determined classification function (f_{K}) and a classification result (K) is determined, and an examination is performed of whether the position (p) at which the stimulus (S_{A}) of the first type is located in the respective test block (BT₁, BT₂, BT₃, BT₄) agrees with the classification result (K), and the number of the test blocks (BT₁, BT₂, BT₃, BT₄) for which agreement is recognised or a value derived from this is used as a measure (M) for the perception capability of a test subject (1) at the time of recording of the additional EEG data (U₁, U₂, U₃, U₄).

2. The method according to claim 1, **characterised in that** based on the same blocks (B₁, B₂, B₃, B₄) determined, the steps c) and d) are performed multiple times, wherein during each individual performance of steps c) and d) respectively
- individual blocks (B₁, B₂, B₃, B₄), are selected, especially according to random criteria, in step c) as calibration blocks (KB₁, KB₂, KB₃, KB₄), wherein this selection especially comprises at least 50% of the blocks (B₁, B₂, B₃, B₄),
- from the other blocks obtained, especially all other blocks, in step d) as test blocks (BT₁, BT₂, BT₃, BT₄) are subjected to the classification function (f_{K}),
- wherein during each performance of steps c) and d) in each case a separate measure is determined for the perception capability or perception quality, and
that an additional measure (M') for the perception capability or perception quality is determined by averaging or aggregation of the separate measures (M₁, M₂, M₃, M₄) contained in the individual executions.

3. The method according to claim 1 or 2, **characterised in that** continuous blocks (B₁, B₂, B₃, B₄) according to step b) are determined, wherein after the recording of one or more blocks (B₁, B₂, B₃, B₄) in each case based on the previously determined blocks (B₁, B₂, B₃, B₄), steps c) and d) are performed, wherein during each individual performance of steps c) and d) respectively
- individual blocks (B₁, B₂, B₃, B₄), are selected, especially according to random criteria, in step c) as calibration blocks (KB₁, KB₂, KB₃, KB₄), wherein this selection especially comprises at least 50% of the blocks (B₁, B₂, B₃, B₄),
- from the other blocks (B₁, B₂, B₃, B₄) determined, especially all other blocks (B₁, B₂, B₃, B₄), in step d) as test blocks (BT₁, BT₂, BT₃, BT₄) are subjected to the classification function (f_{K}),
- wherein during each performance of steps c) and d) in each case a separate measure (MM₁, ..., MMₙ) for the perception capability or perception quality is determined, and
that an examination is performed after the recording of how many blocks (B₁, B₂, B₃, B₄) exceed the separate measure (MM₁, ..., MMₙ) for the perception capacity or perception quality above a predetermined threshold value (T) and the number of blocks (B₁, B₂, B₃, B₄) is determined as an additional measure (M**) for the perception capability or the perception quality, and/or that the mean or median of the individual separate measure (MM₁, ..., MMₙ) is determined as an additional measure (M***) for the perception capability or perception quality.

4. The method according to one of the preceding claims, **characterised in that**
- individual blocks (B₁, B₂, B₃, B₄), are selected, especially according to random criteria, in step c) as calibration blocks (KB₁, KB₂, KB₃, KB₄), wherein this selection especially comprises at least 50% of the blocks (B₁, B₂, B₃, B₄),
- that from the other blocks (B₁, B₂, B₃, B₄) determined, especially all other blocks (B₁, B₂, B₃, B₄), or at least 10% of the other blocks (B₁, B₂, B₃, B₄), in step d) as test blocks (BT₁, BT₂, BT₃, BT₄) are subjected to the classification function (f_{K}), and an examination is conducted to see whether the position (p) at which the stimulus (S_{A}) of the first type is located in the respective block with which the classification result agrees.

5. The method according to one of the preceding claims, **characterised in that** after the formation of the classification function (f_{K}) a number of successively following additional stimuli (S_{A}, S_{B}) is applied to the test subject (1), which according to step b) are combined into additional blocks (B₁', B₂', B₃', B₄') and that for these an individual examination is performed in each case of whether the position (p) at which the stimulus (S_{A}) of the first type is located in the respective additional block agrees with the classification result (K) and the number of additional blocks (B₁', B₂', B₃', B₄') for which agreement is recognised is used as a measure for the perception capability of a test subject (1) at the time of recording of the additional EEG data (U'₁, U'₂, U'₃, U'₄).

6. The method according to one of the preceding claims, **characterised in that**
- at least one of the stimuli (S_{A}, S_{B}), especially all stimuli (S_{A}, S_{B}) or at least stimuli (S_{A}) of the first type, is applied in a vibrotactile manner to the test subject (1),
- that when a classification result exceeding a threshold value is present, an activation stimulus is applied to the test subject (1).

7. The method according to claim 6, **characterized in that** the activation stimulus applied to the test subject (1) consists of an application of functional electrostimulation or using an orthesis, a prosthesis or a robot on an area of the body of the test subject (1), with which the body of the test subject (1) is stimulated at one point or with which part of the body of the test subject (1) body which had undergone vibrotactile stimulation is manipulated.

8. The method according to one of claims 1 to 5, **characterised in that**
- the number of types of stimuli (S_{A}, S_{B}) is predetermined by different sound tones, especially with a different duration, frequency and loudness, in frequencies audible to a human is prespecified, and the respective tone is played for the test subject (1), or
- that the number of types of stimuli (S_{A}, S_{B}) vibration comprises the application of vibration on different body parts and/or with different intensity or duration, applied to the test subject (1) using vibration units.

9. The method according to one of the preceding claims, **characterised in that** the number of types of stimuli (S_{A}, S_{B}) comprises visual stimuli for one eye or both eyes and/or with different intensity and/or duration, which are applied to the test subject (1) by means of a display screen or by means of an illuminant, or that the number of types of stimuli (S_{A}, S_{B}) comprises electrical stimulation on different body parts and/or with different intensity and/or duration, applied to the test subject (1) using electrical stimulators.

10. The method according to one of the preceding claims, **characterised in that** the test subject (1), depending on the respective type of stimulus (S_{A}, S_{B}), is assigned to one of the following cognitive activities:
- Counting or calculating,
- Thinking about movements of body parts, especially extremities of the right or left half of the body, preferably of the arms or hands.

11. The method according to one of the preceding claims, **characterised in that** the classification analyses are performed as follows:
- Discriminant analysis, especially linear discriminant analysis,
- Support vector machines,
- Neural networks.

12. The method according the one of the preceding claims, **characterised in that** after the determination of the perception capability or perception quality, the test subject is brought into various states by cooling or heating the body or part of the body and/or with medication, or that the oxygen partial pressure in the vicinity of the test subject is altered, and
the perception capability and perception quality are repeated according to the same criteria and a new measure is determined for the perception capability and perception quality, and the measure and the new measure are compared.

13. The method according to one of the preceding claims, **characterised in that** after the formation of the classification function (f_{K})
- the test subject is brought into various states by cooling or heating the body or part of the body and/or with medication, or that the oxygen partial pressure in the vicinity of the test subject is altered, and
- several successive additional stimuli (S_{A}, S_{B}) are applied to the test subject (1), which according to step b) are combined into additional blocks (B₁', B₂', B₃', B₄') and that for each of these it is individually investigated whether the position (p) at which the (S_{A}) of the first type is located in the respective additional block agrees with the classification result (K) and the number of additional blocks (B₁', B₂', B₃', B₄') for which agreement is recognised is used as a new measure for the perception capability of a test subject (1) at the time of recording the additional EEG data (U'₁, U'₂, U'₃, U'₄),
- and the measure and the new measure are compared.

14. The method according to one of the preceding claims, **characterised in that** the recorded EEG data are classified using the previously performed classification analysis, and when a determined result of the classification is available, an activation stimulus assigned to this result is applied to the test subject.

## Revendications

1. Méthode pour déterminer la capacité ou la qualité de perception d'une personne (1) au moyen d'une interface cerveau-machine (20),
a) au moins deux types différents l'un de l'autre de stimulations (S_{A}, S_{B}) applicables à une personne (1), en particulier des stimuli acoustiques, mécaniques, électriques ou optique, étant définis, la personne (1) à qui on a notamment demandée d'effectuer à une certaine activité intellectuelle, de préférence, de compter, en présence d'un certain stimulus (S_{A}, S_{B}),
b) une pluralité de stimuli successifs (S_{A}, S_{B}) étant appliqués à la personne (1), et les stimuli (S_{A}, S_{B}) appliqués à la personne (1) étant regroupés en blocs (B₁, B₂, B₃, B₄),
- les blocs (B₁, B₂, B₃, B₄) comprenant un nombre de stimuli successifs (S_{A}, S_{B}) appliqués à la personne (1), dans lesquels respectivement un stimulus (S_{A}) d'un premier type est placé dans une position prédéfinie (p) du bloc (B₁, B₂, B₃, B₄), ainsi que des stimuli (S_{B}) d'un ou de plusieurs autres types sont placés dans les autres positions du bloc (B₁, B₂, B₃, B₄),
- après chacun des stimuli (S_{A}, S_{B}) on constate la réaction de la personne (1) en ce que les données EEG (U₁, U₂, U₃, U₄) de la personne (1) obtenues par le stimulus (S_{A}, S_{B}) ou en relation temporelle avec celui-ci, sont déterminées, et lesdites données EEG (U₁, U₂, U₃, U₄) sont associées au stimulus respectif (S_{A}, S_{B}) ainsi qu'au bloc respectif dans la position concernée (p),
c) à partir des données EEG (U) ainsi déterminées, des données d'étalonnage sont générées par regroupement d'un certain nombre de données EEG déterminées (U₁, U₂, U₃, U₄) et des stimuli (S_{A}, S_{B}) associés auxdites données EEG (U₁, U₂, U₃, U₄) en blocs d'étalonnage (KB₁, KB₂, KB₃, KB₄), dans chacun des blocs d'étalonnage respectifs (KB₁, KB₂, KB₃, KB₄) les données EEG (U₁, U₂, U₃, U₄) de la personne (1) étant associées respectivement à une position lors de l'application d'un premier stimulus (S_{A}, S_{B}) et les données EEG (U₁, U₂, U₃, U₄) de la personne (1) aux autres positions lors de l'application d'un autre stimulus (S_{A}, S_{B}),
- au moyen d'une analyse de classification basée sur des blocs d'étalonnage déterminés (KB₁, KB₂, KB₃, KB₄) une fonction de classification (f_{K}) étant déterminée, qui basée sur les données EEG (U₁, U₂, U₃, U₄) d'un bloc (B₁, B₂, B₃, B₄) de stimuli (S_{A}, S_{B}) indique la position (p) dans laquelle, dans chaque bloc d'étalonnage respectif (KB₁, KB₂, KB₃, KB₄), se trouve le stimulus (S_{A}, S_{B}) du premier type et
d) les données EEG (U₁, U₂, U₃, U₄) d'un certain nombre de blocs test (BT₁, BT₂, BT₃, BT₄) sélectionnés parmi les blocs, sont soumises à la fonction de classification déterminée (f_{K}) et un résultat de classification (K) est déterminé, et on examine si ladite position (p), dans laquelle se trouve le stimulus (S_{A}) du premier type dans le bloc test respectif (BT₁, BT₂, BT₃, BT₄), correspond au résultat de classification (K) et le nombre de blocs test (BT₁, BT₂, BT₃, BT₄) pour lequel une correspondance est détectée ou une valeur dérivant de celui-ci, peut servir de mesure (M) pour la capacité de perception d'une personne (1) au moment de l'enregistrement des autres données EEG (U₁, U₂, U₃, U₄).

2. Méthode selon la revendication 1, **caractérisée en ce que** sur la base des blocs déterminés (B₁, B₂, B₃, B₄) les étapes c) et d) sont réalisées plusieurs fois, à chaque réalisation des étapes c) et d)
- les différents blocs (B₁, B₂, B₃, B₄), étant sélectionnés en particulier selon des critères aléatoires, à l'étape c) en tant que blocs d'étalonnage (KB₁, KB₂, KB₃, KB₄), cette sélection comprenant notamment au moins 50 % des blocs (B₁, B₂, B₃, B₄),
- à partir des autres blocs déterminés, notamment tous les autres blocs, dans l'étape c) en tant que blocs test (BT₁, BT₂, BT₃, BT₄) sont soumis à la fonction de classification (f_{K}),
- lors de chaque réalisation des étapes c) et d) respectivement une mesure séparée est déterminée pour la capacité ou la qualité de perception, et
**en ce qu'**une autre mesure (M') est déterminée pour la capacité ou la qualité de perception par formation d'une valeur moyenne ou agrégation de la mesure (M₁, M₂, M₃, M₄) séparée obtenue dans les différentes réalisations.

3. Méthode selon la revendication 1 ou 2, **caractérisée en ce qu'**en permanence des blocs (B₁, B₂, B₃, B₄) sont déterminés selon l'étape b), après l'enregistrement d'un ou de plusieurs blocs (B₁, B₂, B₃, B₄) les étapes c) et d) étant réalisées respectivement sur la base des blocs déterminés jusqu'à présent (B₁, B₂, B₃, B₄),
lors de la réalisation des étapes c) et d) respectivement
- les différents blocs (B₁, B₂, B₃, B₄), étant sélectionnés en particulier selon des critères aléatoires, à l'étape c) en tant que blocs d'étalonnage (KB₁, KB₂, KB₃, KB₄), cette sélection comprenant notamment au moins 50 % des blocs (B₁, B₂, B₃, B₄),
- à partir des autres blocs déterminés (B₁, B₂, B₃, B₄), notamment tous les autres blocs (B₁, B₂, B₃, B₄), sont soumis à la fonction de classification (f_{K}) dans l'étape d) en tant que blocs test (BT₁, BT₂, BT₃, BT₄),
- lors de chaque réalisation des étapes c) et d) respectivement une mesure séparée (MM₁, ..., MMₙ) étant déterminée pour la capacité ou la qualité de perception, et
**en ce qu'**on analyse, après l'enregistrement dans combien de blocs (B₁, B₂, B₃, B₄) la mesure séparée (MM₁, ..., MMₙ) pour la capacité ou la qualité de perception dépasse une valeur seuil prédéfinie (T) et le nombre des blocs (B₁, B₂, B₃, B₄) en tant qu'autre mesure (M**) est déterminé pour la capacité et la qualité de perception, et/ou **en ce que** la valeur moyenne ou la médiane des différentes mesures séparées (MM₁, ..., MMₙ) est déterminée comme une autre mesure (M***) pour la capacité ou la qualité de perception.

4. Méthode selon une des revendications précédentes, caractérisée en ce
- les différents blocs (B₁, B₂, B₃, B₄), sont sélectionnés en particulier selon des critères aléatoires, à l'étape c) en tant que blocs d'étalonnage (KB₁, KB₂, KB₃, KB₄), cette sélection comprenant notamment au moins 50 % des blocs (B₁, B₂, B₃, B₄),
- en ce qu'à partir des autres blocs déterminés (B₁, B₂, B₃, B₄), notamment tous les autres blocs (B₁, B₂, B₃, B₄) ou au moins 10 % des autres blocs (B₁, B₂, B₃, B₄), dans l'étape d) en tant que blocs test (BT₁, BT₂, BT₃, BT₄) sont soumis à la fonction de classification (f_{K}) et on analyse si chaque position (p), dans laquelle se trouve le stimulus (S_{A}) du premier type dans le bloc respectif, correspond au résultat de classification.

5. Méthode selon une des revendications précédentes, **caractérisée en ce qu'**après la formation de la fonction de classification (f_{K}) un certain nombre d'autres stimuli (S_{A}, S_{B}) successifs sont appliqués à la personne (1), qui sont regroupés à l'étape b) en d'autres groupes (B₁', B₂', B₃', B₄') et **en ce que** pour chacun de ces derniers, on analyse si chaque position (p), dans laquelle se trouve le stimulus (S_{A}) du premier type dans un autre bloc respectif, correspond au résultat de classification (K) et le nombre des autres blocs (B₁', B₂', B₃', B₄'), pour lequel une correspondance est détectée, sert de mesure pour la capacité de perception d'une personne (1) au moment de l'enregistrement des autres données EEG (U'₁, U'₂, U'₃, U'₄).

6. Méthode selon une des revendications précédentes, **caractérisée en ce**
- **qu'**au moins un des stimuli (S_{A}, S_{B}), notamment tous les stimuli (S_{A}, S_{B}) ou au moins les stimuli (S_{A}) du premier type, sont appliqués de manière vibrotactile à la personne (1),
- en ce qu'en présence d'un résultat de classification dépassant une valeur seuil un stimulus d'activation est appliqué à la personne (1),

7. Méthode selon la revendication 6, **caractérisée en ce que** le stimulus d'activation appliquée à la personne (1) consiste en une application de stimulation électrique fonctionnelle ou à l'aide d'une orthèse, d'une prothèse ou d'un robot sur une zone du corps de la personne (1) qui stimule le corps de la personne (1) à un endroit ou qui manipule une partie du corps de la personne (1), qui a été stimulée de manière vibrotactile.

8. Méthode selon une des revendications 1 à 5, **caractérisée en ce que**
- la quantité des types de stimuli (S_{A}, S_{B}) est prédéfinie par des différents sons, en particulier de différentes durée, fréquence et intensité, à des fréquences audibles par un être humain, et le son respectif est émis pour la personne (1), ou
- **en ce que** la quantité des types de stimuli (S_{A}, S_{B}) comprend des expositions à des vibrations de différentes parties du corps et/ou à différente intensité et/ou de différente durée, qui sont appliquées à la personne (1) au moyen d'unités de vibration.

9. Méthode selon une des revendications précédentes, **caractérisée en ce que** la quantité de stimuli (S_{A}, S_{B}) comprend des stimulations pour un oeil ou les deux yeux et/ou à une intensité différente et/ou de différente durée, qui sont appliquées à la personne (1) au moyen d'un écran ou au moyen d'éclairages, ou en ce que la quantité des types de stimuli (S_{A}, S_{B}) comprend des stimulations électriques dans différentes parties du corps et/ou à différente intensité et/ou de différente durée, qui sont appliquées à la personne (1) au moyen de stimulateurs électriques.

10. Méthode selon une des revendications précédentes, **caractérisée en ce qu'**on demande à la personne (1) en fonction de chaque type de stimuli (S_{A}, S_{B}) d'effectuer une des activités intellectuelles suivantes :
- compter ou calculer,
- penser à des mouvements de parties du corps, en particulier les extrémités de la moitié droite ou gauche du corps, de préférence des bras ou des mains.

11. Méthode selon une des revendications précédentes, **caractérisée en ce que** l'analyse de classification s'effectue comme suit :
- analyse discriminante, en particulier analyse discriminante linéaire,
- machines à vecteurs supports,
- réseaux neuronaux.

12. Méthode selon une des revendications précédentes, **caractérisée en ce qu'**après la détermination de la capacité ou de la qualité de perception la personne est amenée dans différents états par refroidissement ou chauffage du corps ou d'une partie du corps et/ou par médication ou **en ce que** la pression partielle d'oxygène est modifiée dans la zone de la personne et
la capacité ou la qualité de perception est répétée selon les mêmes critères et une nouvelle mesure pour la capacité ou la qualité de perception est déterminée, et la mesure et la nouvelle mesure sont comparées l'une à l'autre.

13. Méthode selon une des revendications précédentes, **caractérisée en ce qu'**après la formation de la fonction de classification (f_{K})
- la personne est amenée dans différents états par refroidissement ou par chauffage du corps ou d'une partie du corps et/ou par médication ou **en ce que** la pression partielle d'oxygène de la personne est modifiée, et
- un certain nombre d'autres stimuli (S_{A}, S_{B}) successifs sont appliqués à la personne (1), qui sont regroupés à l'étape b) en d'autres groupes (B₁', B₂', B₃', B₄') et **en ce que** pour chacun de ces derniers, on analyse si chaque position (p), dans laquelle se trouve le stimulus (S_{A}) du premier type dans un autre bloc respectif, correspond au résultat de classification (K) et le nombre des autres blocs (B₁', B₂', B₃', B₄'), pour lequel une correspondante est détectée, sert de mesure pour la capacité de perception d'une personne (1) au moment de l'enregistrement des autres données EEG (U'₁, U'₂, U'₃, U'₄),
- et la mesure et la nouvelle mesure sont comparées l'une à l'autre.

14. Méthode selon une quelconque des revendications précédentes, **caractérisée en ce que** les données EEG enregistrées sont classées au moyen d'un analyse de classification réalisée précédemment, et **en ce qu'**en présence d'un résultat déterminé à partir de la classification un stimulus d'activation associé audit résultat est appliqué à la personne.
